# FASCICULE DE BREVET EUROPEEN

(11) **EP 3 818 205 B1**
(45) Date de publication et mention de la délivrance du brevet: **30.03.2022**
(21) Numéro de dépôt: 19739329.1
(22) Date de dépôt: 27.05.2019
(51) Int. Cl.: D04B 1/14, D04B 1/18, D04B 21/20, A61F 13/08, D04B 1/12, D04B 1/26

(54) **ARTICLE DE COMPRESSION À ENFILAGE/DÉSENFILAGE FACILITÉ ET UTILISATION D'UN FIL POILU DANS LE TRICOTAGE DUDIT ARTICLE**
LEICHT AN-/AUSZIEHBARES KOMPRESSIONSKLEIDUNGSSTÜCK UND VERWENDUNG EINES FADENS MIT FLORFASERN ZUM STRICKEN DES KOMPRESSIONSKLEIDUNGSSTÜCKS
COMPRESSION GARMENT THAT IS EASILY DONNED/DOFFED AND USE OF A THREAD COMPRISING PILE FIBRES FOR KNITTING SAID GARMENT

(30) Priorité: 05.07.2018 FR 1856206
(43) Date de publication de la demande: 12.05.2021
(73) Titulaire: Thuasne, 92300 Levallois Perret (FR)
(72) Inventeur: SCELLES, Hervé, 42170 SAINT JUST SAINT RAMBERT (FR); MOURIER, Bruno, 42380 ABOEN (FR); LABBE, Guillaume, 42350 LA TALAUDIERE (FR); MOTET, Pascal, 42100 SAINT ETIENNE (FR)
(74) Mandataire: Cabinet Beau de Loménie
(86) Numéro de dépôt international: PCT/FR2019/051223
(87) Numéro de publication internationale: WO 2020/008121

(56) Documents cités:
- DE-A1-102012 024 781
- FR-A1- 2 432 867
- JP-A- 2006 219 805
- US-A1- 2004 069 402
- US-A1- 2006 085 894

## Description

### Arrière-plan de l'invention

La présente invention concerne le domaine technique des articles de compression à enfilage/désenfilage amélioré(s).

Les articles de compression, chaussette ou mi-bas, bas, collant ou encore manchon, sont utilisés pour prévenir ou soigner les problèmes de circulation veineuse notamment au niveau du membre inférieur ou supérieur, ainsi que les pathologies liées aux dysfonctionnements du système lymphatique et réduire les œdèmes. Les troubles veineux peuvent avoir plusieurs origines, parmi celles-ci on trouve notamment : une rigidification de la paroi veineuse, une altération des valvules ou encore une augmentation du diamètre des veines.

La pression locale exercée sur un membre par un article à effet compressif est fonction notamment des caractéristiques de force-allongement dudit article.

La pression exercée sur un membre se calcule par la loi de Laplace suivante : P [Pa ou mmHg] = (T [N] X n / (L [m] X R [m]).

P représente la pression exercée en un point donné du membre considéré.

L est la largeur de la zone considérée du membre et n représente le nombre de couches de l'article à effet compressif disposées sur ladite zone.

T est la tension, exprimée en Newtons, exercée par ledit article lorsqu'il est enfilé sur le membre inférieur ou supérieur.

R est le rayon de courbure au point considéré du membre inférieur ou supérieur.

Plus la déficience du système veineux est importante, plus le sang présente des difficultés à refluer depuis la cheville vers le cœur, et plus la pression à exercer au niveau de la cheville est importante.

A titre d'exemple dans le système français, les niveaux de compression peuvent être répartis ainsi :

| | |
|---|---|
| Classe I : 13 à 20 hPa | Classe II : 20,1 à 27 hPa |
| Classe III : 27,1 hPa à 48 hPa | Classe IV : plus de 48 hPa |

Les articles de compression, notamment de classe élevée, sont difficiles à enfiler par le patient, notamment lorsque celui-ci souffre d'une mobilité réduite.

Habituellement, les bas de compression médicaux sont constitués de deux fils, à savoir un fil de trame et un fil dit de maille. Le fil de trame est un fil élastique dont le parcours est quasiment linéaire dans la direction des rangées de mailles des articles de compression. Le fil de trame permet d'attribuer l'effet compressif à l'article de compression. Le fil de maille, également désigné fil de fond, donne à l'article de compression tricoté ses dimensions ainsi que ses propriétés de confort et esthétiques.

Un des critères d'efficacité des articles à effet compressif est la parfaite observance du traitement par les patients. De nombreux patients recherchent des articles à effet compressif au toucher agréable, c'est à dire doux, et faciles à enfiler. La facilité d'enfilage, et de désenfilage, est un paramètre particulièrement important pour les patients souffrant de troubles trophiques, c'est-à-dire par exemple liés à un ulcère veineux, ou un lymphœdème, et/ou ayant des problèmes de motricité.

La recherche de confort et de la facilité d'enfilage ne doit pas cependant se faire au détriment de l'efficacité thérapeutique, en particulier de la compression appliquée, ni de la durabilité de l'article de compression.

On connait ainsi des glisse-pieds se disposant sur les extrémités du pied, et facilitant l'enfilage de l'article de compression à bouts de pied ouverts. Ces glisse-pieds ont pour inconvénients qu'ils sont adaptés uniquement à des articles de compression à bouts de pied ouverts afin de pouvoir retirer les glisse-pieds une fois l'article de compression enfilé.

FR 2.432.867 A1 a pour objet un bas de soutien chirurgical qui peut être placé sur la jambe affectée pour la soutenir avec un traitement compressif, en utilisant deux épaisseurs de tissu superposées dans la zone de compression pour faciliter l'enfilage.

US 2006/085894 A1 a pour objet un vêtement de compression ayant une zone à faible friction réalisée avec l'ajout d'un matériau à faible friction sur la surface intérieure d'un vêtement dans des emplacements discrets pour diminuer la friction entre la peau et le textile, facilitant ainsi l'enfilage.

On connait également des dispositifs supports permettant de placer l'article de compression en tension sur le dispositif support puis d'insérer la jambe du patient à l'intérieur de l'article de compression supporté par ce dispositif.

Les dispositifs précédents sont fastidieux à mettre en œuvre et ne permettent pas le désenfilage. Il existe d'ailleurs des dispositifs médicaux, de type chausse-pied, agencés pour aider spécifiquement au retrait des articles de compression.

On connait également des articles de compression double, c'est-à-dire comprenant une première couche facile à enfiler sans effet compressif, et une seconde couche de compression venant glisser contre la première couche positionnée sur le membre à traiter. Ces articles ont pour inconvénients qu'ils sont plus épais, donc moins esthétiques, et plus chauds. De plus, la seconde couche peut avoir tendance à glisser sur la première couche au porté, l'article de compression n'exerce donc plus correctement l'effet thérapeutique pour lequel il est posé.

EP 2.348.902 B1 a pour objet un vêtement de compression pour la pratique du sport comprenant uniquement sur sa face interne des poils pour améliorer l'isolation thermique. Les techniques de formation des poils utilisées ne permettent de former des poils que sur une seule face du textile, (rasage des boucles ou découpe des fils de liage joignant deux textiles superposés pour un textile velours). Les poils sont ainsi formés sur la face interne du textile.

Néanmoins, s'agissant des articles de compression médicaux, il est recherché au contraire que ces derniers soient légers, respirants et n'apportent pas de fonction d'isolation thermique supplémentaire puisqu'ils sont portés généralement sous les vêtements.

### Objet et résumé de l'invention

La présente invention vise à proposer un article de compression, en particulier médical, dont l'enfilage et le désenfilage sont facilités, les moyens facilitant l'enfilage et le désenfilage étant agencés directement dans l'article de compression sans besoin d'un dispositif externe complexe à mettre en œuvre.

La présente invention vise à proposer un article de compression dont le confort au porté est amélioré, en particulier le toucher, notamment de la face interne de l'article de compression venant en contact avec la peau.

La présente invention a pour objet, selon un premier aspect, un article de compression à enfilage et/ou désenfilage facilité(s) comprenant une pièce tricotée de compression comprenant au moins un fil de fond maillant et au moins un fil élastique, notamment tramé.

Avantageusement, la pièce tricotée comprend au moins un fil poilu, tricoté dans la pièce tricotée, en sorte que la pièce tricotée présente, selon, tout ou partie, de sa face interne et de sa face externe, des poils se projetant de ces dernières, lesdits poils étant configurés en sorte de s'orienter dans la direction d'enfilage et/ou de désenfilage de la pièce tricotée.

Les inventeurs ont découvert que les poils adoptent naturellement une disposition correspondant à la direction d'enfilage ou de désenfilage, et s'aplatissent sur la face interne de la pièce tricotée, créant une face interne dont la surface est lisse. La surface lisse de la face interne de la pièce tricotée présente donc un faible coefficient de frottement.

Le tricotage d'un fil poilu implique la disposition d'une multiplicité de poils indifféremment sur les faces interne et externe.

On comprend par fil poilu tricoté tout fil comprenant des poils se projetant sur sa longueur et configuré pour pouvoir être mis en œuvre sur un métier à tricoter. En particulier, le fil tricoté poilu peut être un fil formant des mailles (bouclées et/ou chargées) et/ou un fil tramé.

De préférence, le fil poilu est distinct dudit fil de fond maillant et/ou dudit fil de trame.

De préférence, le fil poilu tricoté n'est pas élastique.

On comprend dans le présent texte par fil non élastique, tout fil ayant un allongement à rupture inférieur ou égal à 100%, de préférence inférieur ou égal à 50%, encore de préférence inférieur ou égal à 30%.

La pièce tricotée comprend des rangées de mailles, notamment disposées dans le sens transverse T de la pièce tricotée, et des colonnes de mailles, notamment disposées dans le sens longitudinal L de la pièce tricotée.

On comprend par fil de fond maillant ou fil maillant, tout fil tricoté formant des mailles bouclées et/ou des mailles chargées.

On comprend par fil tramé, un fil inséré dans les rangées de mailles de la pièce tricotée, en particulier sans former de mailles bouclées, et de préférence en réalisant des flottés et/ou des charges.

On désigne indifféremment dans le présent texte par article ou pièce tricotée de compression, tout article ou pièce tricotée à effet compressif, ou de contention.

On désigne par article de compression comprenant une pièce tricotée de compression, tout article dont la pièce tricotée comprend au moins une portion tubulaire destinée à être disposée autour d'au moins une portion d'un membre sur lequel la compression doit être exercée.

La pièce tricotée peut être obtenue par l'assemblage, par exemple par couture, de différents panneaux tricotés, ou être une pièce tricotée unitaire.

La portion tubulaire peut être obtenue d'un seul tenant sans couture (notamment par le tricotage d'une portion de tube) ou est formée par assemblage des bords correspondants de la pièce tricotée unitaire, par exemple par couture ou par tricotage sur le métier à tricoter.

Lorsque la pièce tricotée comprend une pointe de pied, cette dernière peut être fermée lors du tricotage ou par couture.

La pièce tricotée est ainsi de préférence choisie parmi : un bas, un mi-bas, une chaussette, un collant, un manchon pour l'avant-bras ou le bras, ou une combinaison de ces derniers.

Avantageusement, la formation des poils ne dépend pas d'une technique de tricotage particulière. La pièce tricotée selon l'invention peut ainsi être tricotée sur un métier à tricoter rectiligne ou circulaire.

La pièce tricotée comprend des faces interne et externe opposées, la face interne étant destinée en fonctionnement à venir contre la peau.

Avantageusement, le fil poilu est fabriqué par tricotage avant l'étape de tricotage de la pièce tricotée.

Dans un mode de réalisation, le fil de fond maillant a un titre supérieur ou égal à 50 dtex et inférieur ou égal à 650 dtex.

Dans un mode de réalisation, la pièce tricotée comprend un fil de fond maillant élastique, et éventuellement un fil de fond maillant non élastique.

On comprend par fil élastique dans le cadre de la présente invention, s'agissant notamment du fil de fond maillant élastique et/ou du fil de trame (notamment l'âme du fil de trame), tout fil ayant un allongement à rupture supérieur ou égal à 300%, de préférence supérieur ou égal à 400%, encore de préférence supérieur ou égal à 500%.

L'allongement à rupture peut être déterminé par exemple à l'aide de la norme ISO 2062:2009.

Dans une variante, le fil poilu comprend une âme et des poils se projetant de ladite âme.

Le fil présente ainsi avantageusement une bonne résistance mécanique. De plus, il est possible d'ajuster la longueur (mm) et la densité de poils (nombre de poils/mètre linéaire d'âme ou de fil poilu) sur l'âme.

Le fil poilu selon l'invention est ainsi un assemblage d'une âme et d'une couverture de poils se projetant de cette dernière procurant un effet velours aux faces interne et externe de la pièce tricotée.

L'âme du fil poilu, et/ou, les poils du fil poilu, et/ou, le fil de maille de fond, et/ou, le ou les fil(s) de couverture de l'âme élastique du fil de trame lorsqu'il est élastique, comprend/comprennent un ou plusieurs fil(s) multifilamentaire(s) et/ou fil(s) filé(s) de fibres dont le ou les matériaux le/les constituant est/sont choisi(s) parmi une liste I constituée de matériaux synthétiques et une liste II constituée de matériaux naturels ou artificiels.

La liste I est ainsi constituée des matériaux suivants : les polyesters, en particulier le polyéthylène téréphtalate ; les polyamides, en particulier le polyamide 6-6 ; les polyoléfines, en particulier le polypropylène et le polyéthylène, ou leur mélange ; de préférence en polyéthylène téréphtalate.

La liste II est ainsi constituée des matériaux suivants : le coton, le lin, la cellulose régénérée, ou leur mélange ; en particulier la viscose et/ou la laine.

Avantageusement, la construction du fil poilu permet que les poils et l'âme soient dans des fils de structure et dans des matériaux différents, ce qui permet de différencier les fonctions des poils de la structure de l'âme.

De préférence, les poils sont répartis dans des tronçons ou segments de fils se projetant selon l'âme du fil poilu, encore de préférence ces tronçons comprennent/sont constitués, notamment chacun, d'une première extrémité solidarisée à l'âme du fil poilu, et d'une seconde extrémité libre configurée pour adopter une direction correspondant au sens d'enfilage ou de désenfilage.

Ainsi, un tronçon de fil peut comprendre une pluralité de poils, dont le nombre et le titre individuel sont fonction du nombre et du titre individuel de chacune des fibres et/ou de chacun des filaments que comprend le fil utilisé pour le tronçon.

Dans une variante, l'âme du fil poilu est tricotée.

Cette disposition améliore sa solidité, notamment sa résistance mécanique.

De préférence, le ou les fils à partir duquel ou desquels les poils sont formés, sont ancrés lors du tricotage du fil poilu, notamment en même temps que lors du tricotage de l'âme.

Dans une variante, l'âme est une chainette dans laquelle les poils sont ancrés.

La chainette est par définition en mailles jetées et donc indémaillable, ce qui améliore encore la résistance mécanique du fil poilu et la résistance à l'arrachement des poils dans l'âme tricotée.

Dans une variante, l'âme du fil poilu est tricotée avec un ou plusieurs fil(s) multifilamentaire(s) ou fil(s) filé(s) de fibres, ou leur mélange.

Dans une variante, le nombre de tronçons de poils par centimètre mesuré sur la longueur du fil poilu est supérieur ou égal à 3, de préférence inférieur ou égal à 20, de préférence inférieur ou égal à 15, plus préférentiellement inférieur ou égal à 10.

La densité en poils doit être suffisante pour faciliter l'enfilage/désenfilage, mais tout de même limitée, car la fonction d'isolation thermique n'est pas recherchée.

Dans une variante, la pièce tricotée comprend :
a. au moins une rangée de mailles T comprenant au moins un fil élastique tramé,
b. au moins deux rangées de mailles M1 et M2, chacune comprenant au moins un fil poilu formant des mailles, ladite au moins une rangée de mailles T étant disposée entre les rangées M1 et M2 adjacentes, et la longueur d'au moins 80% en nombre des poils dudit au moins un fil poilu dans au moins l'une des rangées de mailles M1 et M2 est supérieure ou égale à la distance (d) séparant les rangées de mailles M1 et M2.

Avantageusement, les poils recouvrent le fil élastique tramé ce qui permet de diminuer, voire de supprimer, l'effet de cisaillement apporté par le fil élastique tramé puisque les poils vont venir se placer entre la peau et la zone de la pièce tricotée comprenant le fil élastique tramé.

L'effet de cisaillement est en effet assuré principalement par les fils de trame élastiques, lequel effet de cisaillement est un frein à l'enfilage et au désenfilage.

Dans une variante, la longueur d'au moins 80% en nombre des poils par centimètre mesurée sur la longueur du fil poilu est supérieure ou égale à 1 mm, de préférence supérieure ou égale à 5 mm, encore de préférence supérieure ou égale à 10 mm, en particulier inférieure ou égale à 30 mm, par exemple de l'ordre de 2 mm +/- 7%.

Dans un mode de réalisation, la longueur d'au moins 80% en nombre des poils par centimètre mesurée sur la longueur du fil poilu est inférieure ou égale à 10 mm.

Cette mesure peut être effectuée par observations à l'aide d'un microscope, et est basée sur la moyenne d'au moins dix mesures effectuées en trois régions différentes sur la longueur du fil poilu et dont la longueur est chacune d'un centimètre.

Dans une variante, le fil poilu est fabriqué par tricotage sur un métier à tricoter à crochet.

Cette technique permet de former une âme indémaillable dans laquelle les poils sont ancrés lors du tricotage du fil poilu et ainsi de faire varier à souhait la longueur et la densité des poils.

Dans une variante, le fil poilu forme des mailles, notamment des mailles chargées et/ou des mailles bouclées.

Dans une variante, la pièce tricotée comprend un fil poilu tramé.

Dans une variante, le fil élastique comprend une âme élastique et un ou plusieurs fil(s) de couverture, et le fil poilu est un fil choisi parmi ledit ou lesdits fil(s) de couverture.

Dans une variante, le fil élastique comprend une âme élastique et un ou plusieurs fil(s) de couverture, et l'âme a un titre inférieur ou égal à 740 dtex.

De préférence, l'âme élastique a un titre supérieur ou égal à 200 dtex, encore de préférence supérieur ou égal à 350 dtex, préférentiellement supérieur ou égal à 500 dtex.

Dans une variante, le fil poilu a un titre supérieur ou égal à 15 000 Nm, en particulier supérieur ou égal à 20 000 Nm, en particulier inférieur ou égal à 30 000 Nm.

Le fil poilu doit avoir une finesse adaptée avec la jauge de la pièce tricotée.

Dans un mode de réalisation, le fil poilu a un titre supérieur ou égal à 30 000 Nm, et éventuellement inférieur ou égal à 100 000 Nm.

Dans ce cas, la pièce tricotée présente une jauge plus importante que celle qu'elle présente lorsque le fil poilu a une finesse inférieure à 30 000 Nm.

La présente invention a pour objet l'utilisation d'un fil poilu, pour faciliter l'enfilage et/ou le désenfilage, dans le tricotage d'une pièce tricotée de compression pour la fabrication d'un article de compression, ladite pièce tricotée comprenant au moins un fil de fond maillant et au moins un fil élastique, notamment tramé, ledit au moins un fil poilu est tricoté dans la pièce tricotée en sorte que la pièce tricotée présente, selon, tout ou partie, de sa face interne et de sa face externe, des poils se projetant de ces dernières, lesdits poils étant agencés en sorte de s'orienter dans la direction d'enfilage ou de désenfilage de la pièce tricotée.

L'article de compression reproduit, et donc ledit au moins un fil poilu et la pièce tricotée reproduisent, indifféremment l'une quelconque des variantes de réalisation ou définitions décrite(s) en référence à un premier aspect de l'invention.

Dans une variante, la pièce tricotée est réalisée sur un métier à tricoter circulaire ou sur un métier à tricoter rectiligne, et le fil poilu est fabriqué lors d'une étape de tricotage, en particulier sur un métier à tricoter à crochet, préalablement à l'étape de tricotage de la pièce tricotée.

### Description des figures

La présente invention sera mieux comprise à la lecture de la description d'exemples de réalisation selon l'invention cités à titre non limitatif et illustrés par les figures suivantes et dans lesquelles :
- la figure **1** est une représentation schématique vue de face d'un premier exemple d'article de compression selon l'invention ;
- la figure **2** est une représentation d'une photographie d'un exemple de fil poilu selon l'invention ;
- les figures **3** à **5** sont des représentations de trois exemples de schéma de mailles de la pièce tricotée de l'article représenté à la figure **1** ;
- la figure **6** est une représentation du schéma de mailles représenté à la figure **3**, répétée deux fois.

### Description détaillée de l'invention

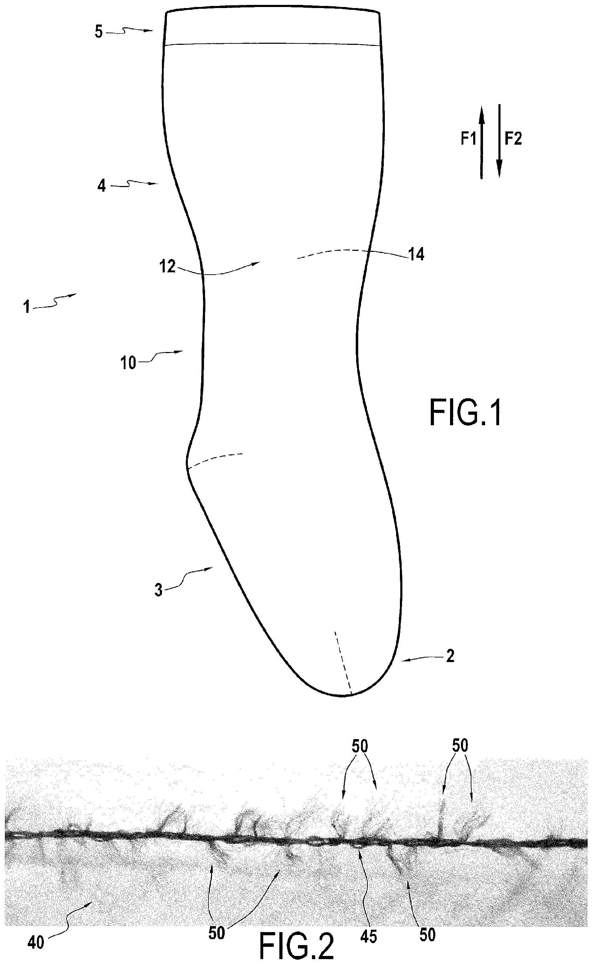

L'article de compression **1** à enfilage et/ou désenfilage facilité(s) représenté à la figure **1** est une chaussette de compression **1** comprenant une pièce tricotée **10** de compression, réalisée intégralement par tricotage.

Cette pièce tricotée **10** comprend une pointe de pied **2**, de préférence fermée après le tricotage (par exemple par couture), une partie de pied tricotée **3**, et dans son prolongement une partie correspondant à la jambe **4** tricotée se terminant par un revers **5**.

La pièce tricotée **10** comprend un fil de fond maillant **20**, notamment élastique, un fil élastique tramé **30**, et un fil poilu **40** tricoté dans la pièce tricotée **10** en sorte que la pièce tricotée **10** présente, selon tout ou partie de sa face interne **12** et de sa face externe **14**, des poils **50** se projetant de ces dernières, lesdits poils **50** étant configurés en sorte de s'orienter dans la direction d'enfilage **F1** et/ou de désenfilage **F2** de la pièce tricotée **10**. Les poils **50** sont répartis dans des tronçons ou segments de fils se projetant selon l'âme **45** du fil poilu **40**. De préférence, ces tronçons comprennent/sont constitués chacun d'une première extrémité solidarisée à l'âme **45** du fil poilu, et d'une seconde extrémité libre configurée pour adopter une direction correspondant au sens d'enfilage ou de désenfilage. Le fil poilu **40** comprend une âme **45** et des poils **50** se projetant de ladite âme **45**. L'âme **45** est tricotée, de préférence, il s'agit d'une chainette dans laquelle les poils **50** sont ancrés. L'âme **45** du fil poilu **40** est tricotée avec des fils multifilamentaires et/ou des fils filés de fibres, chacun en polyamide ayant un titre de l'ordre de 44 dtex. Les poils **50** ancrés lors du tricotage du fil poilu **40** sur un métier à crochet sont formés par des fils en viscose dont le titre de chacun est de l'ordre de 44 dtex. Le fil poilu **40** a un titre de l'ordre de 23 000 Nm.

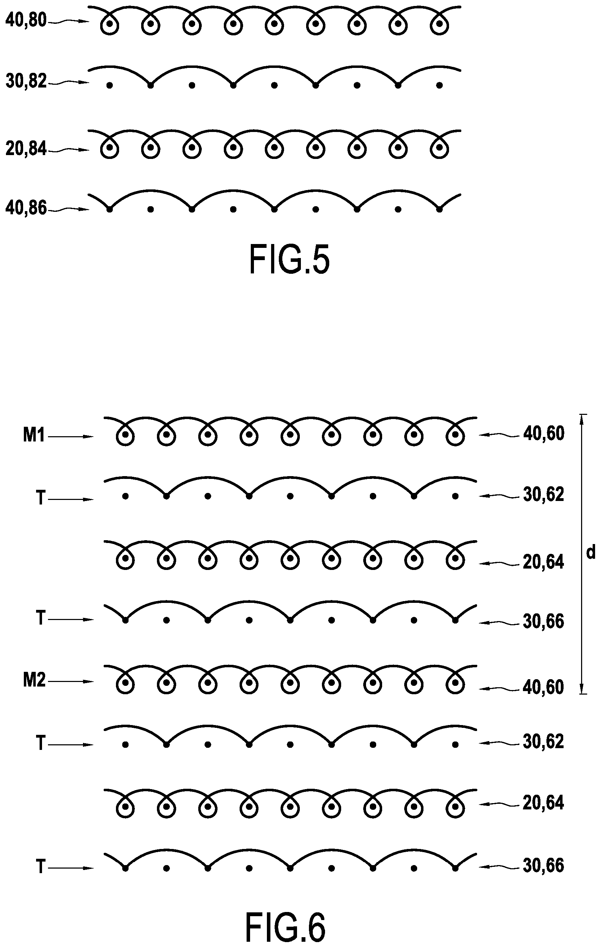

Le nombre de tronçons de poils **50** par centimètre mesuré sur la longueur du fil poilu **40** est supérieur ou égal à 3 et inférieur ou égal à 10, en particulier de l'ordre de 4. Le fil de trame élastique **30** comprend une âme élastique en élasthanne dont le titre est de l'ordre de 620 dtex, et deux fils de couverture guipés en polyamide dont chacun a un titre de l'ordre de 22 dtex. Un premier fil de couverture est guipé dans le sens S tandis que le second fil de couverture est guipé dans le sens Z. Enfin, la pièce tricotée **10** comprend un fil élastique tramé **30** disposé dans une rangée de mailles T, tel que cela est illustré sur la figure **6**, entre deux rangées de mailles **M1** et **M2**, chacune comprenant au moins un fil poilu **40** formant des mailles, notamment envers sur toutes les aiguilles. La rangée de mailles **T** est disposée entre les rangées **M1** et **M2** adjacentes, et la longueur d'au moins 80% des poils **50** dudit au moins un fil poilu **40** dans l'une des rangées de mailles **M1** et **M2** est supérieure ou égale à la distance **d** séparant les rangées de mailles **M1** et **M2.** La distance **d** représentée à la figure **6** de manière schématique s'évalue non pas sur le métier à tricoter mais sur la pièce tricotée **10** relaxée, en particulier dans les conditions hygrométriques et de températures définies dans la norme NF ISO 139 :2005. Les poils **50** ont ainsi, pour au moins 80% en nombre, de préférence au moins 95% en nombre, par exemple une longueur de l'ordre de 2 mm +/- 7%. De préférence, le fil poilu **40** forme des mailles, notamment des mailles chargées et/ou des mailles bouclées, et n'est pas tramé. Enfin, le fil de fond maillant **20** est élastique et comprend ainsi une âme en élasthanne dont le titre est de l'ordre de 44 dtex et deux fils de couverture, notamment en polyamide, en particulier guipés, dont chacun à un titre de l'ordre 78 dtex.

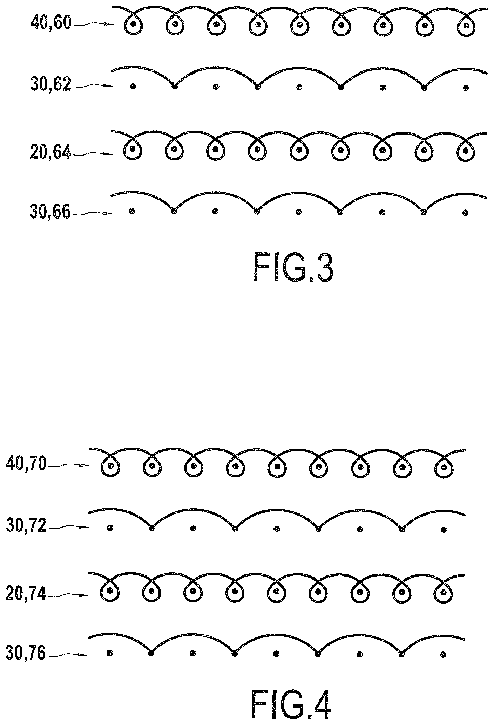

Le premier exemple de schéma de mailles de la figure **3** comprend ainsi une première rangée de mailles jersey **60**, notamment envers, tricotées sur toutes les aiguilles avec le fil poilu **40**, une seconde rangées **62** de mailles chargées une aiguille sur deux tricotée avec le fil de trame élastique **30**, une troisième rangée **64** de mailles jersey, notamment envers, tricotées sur toutes les aiguilles avec le fil de fond maillant **20** élastique, et enfin une quatrième rangées de mailles **66** chargées une aiguille sur deux, en quinconce avec la seconde rangées de mailles **62** chargées, et tricotées avec le fil de trame élastique **30**. Ce schéma de mailles correspond en particulier à la structure compressive allant du dessous de la malléole jusqu'au-dessous du revers **5** de l'article de compression **1**, c'est-à-dire sensiblement à la partie de jambe **4**.

Le second exemple de schéma de mailles de la figure **4** comprend une première rangée de mailles jersey **70**, notamment envers, tricotées sur toutes les aiguilles avec le fil poilu **40**, une seconde rangée **72** de mailles chargées une aiguille sur deux tricotées avec le fil de trame élastique **30**, une troisième rangée **74** de mailles jersey, notamment envers, tricotées sur toutes les aiguilles avec le fil de fond maillant **20** élastique, et enfin une quatrième rangées de mailles **76** chargées une aiguille sur deux, identique à la seconde rangées de mailles **72**, et tricotées avec le fil de trame élastique **30**. Ce schéma de mailles correspond en particulier à une structure côtelée de l'article de compression **1** attribuée par le fil de trame, en particulier disposée au niveau de la structure compressive allant du dessous de la malléole jusqu'au-dessous du revers **5** de l'article **1**, c'est-à-dire sensiblement à la partie de jambe **4**.

Le troisième exemple de schéma de mailles de la figure **5** comprend une première rangée de mailles jersey **80**, notamment envers, tricotées sur toutes les aiguilles avec le fil poilu **40**, une seconde rangée **82** de mailles chargées une aiguille sur deux tricotée avec le fil de trame élastique **30**, une troisième rangée **84** de mailles bouclées une aiguille sur deux, notamment envers, dans le fil de fond maillant **20** élastique, et enfin une quatrième rangée de mailles **86** chargées une aiguille sur deux, en quinconce avec la seconde rangée de mailles chargées **82**, et tricotées avec le fil de trame élastique **30**. Ce schéma de mailles correspond en particulier à une structure côtelée de l'article de compression **1** attribuée par le fil de fond maillant élastique **20**, en particulier disposée au niveau de la structure compressive allant du dessous de la malléole jusqu'au-dessous du revers **5** de l'article **1**, (c'est-à-dire correspondant sensiblement à la partie de jambe **4**).

Avantageusement, le schéma de mailles, la densité et la longueur des poils **50** permettent de déterminer avec précision l'effet « velours » recherché pour la pièce tricotée **10**, et donc d'impacter l'effet de cisaillement des fils élastiques tramés **30**, et par conséquence de faciliter l'enfilage et le désenfilage de l'article de compression **1**. Il est également possible de combiner cet effet avec différentes structures tricotées, par exemple avec un effet côtelé tel que décrit ci-avant.

## Revendications

1. Article de compression (1) à enfilage et/ou désenfilage facilité(s) comprenant une pièce tricotée de compression (10) comprenant au moins un fil de fond maillant (20) et au moins un fil élastique, notamment tramé (30), **caractérisé en ce que** la pièce tricotée (10) comprend au moins un fil poilu (40) tricoté dans la pièce tricotée (10) en sorte que la pièce tricotée (10) présente, selon tout ou partie de sa face interne (12) et de sa face externe (14), des poils (50) se projetant de ces dernières, lesdits poils (50) étant configurés en sorte de s'orienter dans la direction d'enfilage (F1) et/ou de désenfilage (F2) de la pièce tricotée (10).

2. Article de compression (1) selon la revendication 1, **caractérisé en ce que** le fil poilu (40) comprend une âme (45) et des poils (50) se projetant de ladite âme (45).

3. Article de compression (1) selon la revendication 2, **caractérisé en ce que** l'âme (45) est tricotée.

4. Article de compression (1) selon l'une ou l'autre des revendications 2 et 3, **caractérisé en ce que** l'âme (45) est une chainette dans laquelle les poils (50) sont ancrés.

5. Article de compression (1) selon l'une quelconque des revendications 2 à 4, **caractérisé en ce que** l'âme (45) du fil poilu (40) est tricotée avec un ou plusieurs fils multifilamentaires ou fils filés de fibres ou leur mélange.

6. Article de compression (1) selon l'une quelconque des revendications 1 à 5, **caractérisé en ce que** le nombre de tronçons (55) de poils (50) par centimètre mesuré sur la longueur du fil poilu (40) est supérieur ou égal à 3, encore de préférence inférieur ou égal à 20.

7. Article de compression (1) selon l'une quelconque des revendications 1 à 6, **caractérisé en ce que** la pièce tricotée comprend :
a. au moins une rangée de mailles T (62,66,72,76,82) comprenant au moins un fil élastique tramé (30),
b. au moins deux rangées de mailles M1 et M2 (60,70,80,86), chacune comprenant au moins un fil poilu (40) formant des mailles, ladite au moins une rangée de mailles T étant disposée entre les rangées M1 et M2 adjacentes, et **en ce que** la longueur d'au moins 80% en nombre des poils (50) dudit au moins un fil poilu (40) dans l'une des rangées de mailles M1 et M2 (60,70,80,86) est supérieure ou égale à la distance (d) séparant les rangées de mailles M1 et M2.

8. Article de compression (1) selon l'une quelconque des revendications 1 à 7, **caractérisé en ce que** la longueur d'au moins 80% en nombre des poils (50) par centimètre, mesurée sur la longueur du fil poilu (40), est supérieure ou égale à 1 mm, de préférence supérieure ou égale à 5 mm, encore de préférence supérieure ou égale à 10 mm.

9. Article de compression (1) selon l'une quelconque des revendications 1 à 8, **caractérisé en ce que** le fil poilu (40) est fabriqué par tricotage sur un métier à tricoter à crochet.

10. Article de compression (1) selon l'une quelconque des revendications 1 à 9, **caractérisé en ce que** le fil poilu (40) forme des mailles, notamment des mailles chargées et/ou des mailles bouclées.

11. Article de compression selon quelconque des revendications 1 à 10, **caractérisé en ce que** la pièce tricotée comprend un fil poilu tramé.

12. Article de compression selon l'une quelconque des revendications 1 à 11, **caractérisé en ce que** le fil élastique comprend une âme élastique et un ou plusieurs fil(s) de couverture, et **en ce que** le fil poilu est un fil choisi parmi ledit ou lesdits fil(s) de couverture.

13. Article de compression selon l'une quelconque des revendications 1 à 12, **caractérisé en ce que** le fil élastique comprend une âme élastique et un ou plusieurs fil(s) de couverture, et **en ce que** l'âme a un titre inférieur ou égal à 740 dtex.

14. Article de compression (1) selon l'une quelconque des revendications 1 à 13, **caractérisé en ce que** le fil poilu (40) a un titre supérieur ou égal à 15 000 Nm, en particulier supérieur ou égal à 20 000 Nm.

15. Utilisation d'un fil poilu (40), pour faciliter l'enfilage et/ou le désenfilage, dans le tricotage d'une pièce tricotée (10) de compression pour la fabrication d'un article de compression (1), ladite pièce tricotée (10) comprenant au moins un fil de fond maillant (20) et au moins un fil élastique, notamment tramé (30), ledit au moins un fil poilu (40) est tricoté dans la pièce tricotée (10) en sorte que la pièce tricotée (10) présente selon, tout ou partie, de sa face interne (12) et de sa face externe (14), des poils (50) se projetant de ces dernières, lesdits poils (50) étant agencés en sorte de s'orienter dans la direction d'enfilage (F1) et/ou de désenfilage (F2) de la pièce tricotée (10).

16. Utilisation selon la revendication 15, **caractérisée en ce que** la pièce tricotée (10) est tricotée sur un métier à tricoter circulaire ou sur un métier à tricoter rectiligne, et **en ce que** le fil poilu (40) est fabriqué lors d'une étape de tricotage, en particulier sur un métier à tricoter à crochet, préalablement à l'étape de tricotage de la pièce tricotée (10).

## Patentansprüche

1. Leicht an- und/oder ausziehbarer Kompressionsartikel (1), umfassend ein gestricktes Kompressionsteil (10), das wenigstens einen Maschengrundfaden (20) und wenigstens einen elastischen Faden, insbesondere Schussfaden (30) umfasst, **dadurch gekennzeichnet, dass** das gestrickte Teil (10) wenigstens einen Florfaden (40) umfasst, der in das gestrickte Teil (10) gestrickt ist, so dass das gestrickte Teil (10) entlang seiner gesamten Innenseite (12) und seiner gesamten Außenseite (14) oder einem Teil dieser Fasern (50) aufweist, die von letzteren abstehen, wobei die Fasern (50) derart ausgebildet sind, dass sie sich in Anzieh- (F1) und/oder Ausziehrichtung (F2) des gestrickten Teils (10) ausrichten.

2. Kompressionsartikel (1) nach Anspruch 1, **dadurch gekennzeichnet, dass** der Florfaden (40) eine Seele (45) und Fasern (50) aufweist, die von der Seele (45) abstehen.

3. Kompressionsartikel (1) nach Anspruch 2, **dadurch gekennzeichnet, dass** die Seele (45) gestrickt ist.

4. Kompressionsartikel (1) nach dem einen oder anderen der Ansprüche 2 und 3, **dadurch gekennzeichnet, dass** die Seele (45) eine Kette ist, in der die Fasern (50) verankert sind.

5. Kompressionsartikel (1) nach einem der Ansprüche 2 bis 4, **dadurch gekennzeichnet, dass** die Seele (45) des Florfadens (40) mit einem oder mehreren Multifilamentfäden oder Faserspinnfäden oder deren Mischung gestrickt ist.

6. Kompressionsartikel (1) nach einem der Ansprüche 1 bis 5, **dadurch gekennzeichnet, dass** die Anzahl von Abschnitten (55) von Fasern (50) pro Zentimeter, gemessen über die Länge des Florfadens (40), größer als oder gleich 3 ist, weiterhin bevorzugt kleiner als oder gleich 20 ist.

7. Kompressionsartikel (1) nach einem der Ansprüche 1 bis 6, **dadurch gekennzeichnet, dass** das gestrickte Teil umfasst:
a. wenigstens eine Reihe von Maschen T (62, 66, 72, 76, 82), die wenigstens einen elastischen Schussfaden (30) umfasst,
b. wenigstens zwei Reihen von Maschen M1 und M2 (60, 70, 80, 86), wobei eine jede wenigstens einen Maschen bildenden Florfaden (40) umfasst, wobei die wenigstens eine Reihe von Maschen T zwischen den benachbarten Reihen M1 und M2 angeordnet ist, und dass die Länge von wenigstens 80 % an Anzahl Fasern (50) des wenigstens einen Florfadens (40) in einer der Maschenreihen M1 und M2 (60, 70, 80, 86) größer als der oder gleich dem Abstand (d) zwischen den Maschenreihen M1 und M2 ist.

8. Kompressionsartikel (1) nach einem der Ansprüche 1 bis 7, **dadurch gekennzeichnet, dass** die Länge von wenigstens 80 % an Anzahl Fasern (50) pro Zentimeter, gemessen über die Länge des Florfadens (40), mehr als oder gleich 1 mm, bevorzugt mehr als oder gleich 5 mm, weiterhin bevorzugt mehr als oder gleich 10 mm beträgt.

9. Kompressionsartikel (1) nach einem der Ansprüche 1 bis 8, **dadurch gekennzeichnet, dass** der Florfaden (40) durch Stricken auf einer Hakenstrickmaschine hergestellt ist.

10. Kompressionsartikel (1) nach einem der Ansprüche 1 bis 9, **dadurch gekennzeichnet, dass** der Florfaden (40) Maschen, insbesondere Doppelmaschen und/oder Schlingenmaschen, bildet.

11. Kompressionsartikel nach einem der Ansprüche 1 bis 10, **dadurch gekennzeichnet, dass** das gestrickte Teil einen Schussflorfaden umfasst.

12. Kompressionsartikel nach einem der Ansprüche 1 bis 11, **dadurch gekennzeichnet, dass** der elastische Faden eine elastische Seele und einen Deckfaden oder mehrere Deckfäden umfasst und dass der Florfaden ein Faden ist, der aus dem Deckfaden/den Deckfäden ausgewählt ist.

13. Kompressionsartikel nach einem der Ansprüche 1 bis 12, **dadurch gekennzeichnet, dass** der elastische Faden eine elastische Seele und einen Deckfaden oder mehrere Deckfäden umfasst, und dass die Seele eine Feinheit von weniger als oder gleich 740 dtex aufweist.

14. Kompressionsartikel (1) nach einem der Ansprüche 1 bis 13, **dadurch gekennzeichnet, dass** der Florfaden (40) eine Feinheit von mehr als oder gleich 15.000 Nm, insbesondere von mehr als oder gleich 20.000 Nm aufweist.

15. Verwendung eines Florfadens (40) zum Erleichtern des Anziehens und/oder Ausziehens, beim Stricken eines gestrickten Kompressionsteils (10) für die Herstellung eines Kompressionsartikels (1), wobei das gestrickte Teil (10) wenigstens einen Maschengrundfaden (20) und wenigstens einen elastischen Faden, insbesondere Schussfaden (30) umfasst, wobei der wenigstens eine Florfaden (40) in das gestrickte Teil (10) gestrickt ist, so dass das gestrickte Teil (10) entlang seiner gesamten Innenseite (12) und seiner gesamten Außenseite (14) oder einem Teil dieser Fasern (50) aufweist, die von letzteren abstehen, wobei die Fasern (50) derart angeordnet sind, dass sie sich in Anzieh- (F1) und/oder Ausziehrichtung (F2) des gestrickten Teils (10) ausrichten.

16. Verwendung nach Anspruch 15, **dadurch gekennzeichnet, dass** das gestrickte Teil (10) auf einer Rundstrickmaschine oder auf einer Flachstrickmaschine gestrickt wird, und dass der Florfaden (40) während eines Strickschrittes, insbesondere auf einer Hakenstrickmaschine, vor dem Schritt des Strickens des gestrickten Teils (10) hergestellt wird.

## Claims

1. A compression garment (1) that is easy to slip on and/or to slip off comprising a compression knitted piece (10) comprising at least one knitting ground yarn (20) and at least one elastic yarn, in particular inlaid yarn (30), **characterized in that** the knitted piece (10) comprises at least one pile yarn (40) knitted into the knitted piece (10) so that the knitted piece (10) has, along all or part of its inner face (12) and of its outer face (14), pile strands (50) projecting from the latter, said pile strands (50) being configured so as to be oriented in the direction in which the knitted piece (10) is slipped on (F1) and/or off (F2).

2. The compression garment (1) as claimed in claim 1, **characterized in that** the pile yarn (40) comprises a core (45) and pile strands (50) projecting from said core (45).

3. The compression garment (1) as claimed in claim 2, **characterized in that** the core (45) is knitted.

4. The compression garment (1) as claimed in either of claims 2 and 3, **characterized in that** the core (45) is a pillar stitch in which the pile strands (50) are anchored.

5. The compression garment (1) as claimed in any one of claims 2 to 4, **characterized in that** the core (45) of the pile yarn (40) is knitted with one or more multifilament yarns or spun yarns of fibers or a mixture thereof.

6. The compression garment (1) as claimed in any one of claims 1 to 5, **characterized in that** the number of sections (55) of pile strands (50) per centimeter measured along the length of the pile yarn (40) is greater than or equal to 3, preferably still less than or equal to 20.

7. The compression garment (1) as claimed in any one of claims 1 to 6, **characterized in that** the knitted piece comprises:
a. at least one row of stitches T (62,66,72,76,82) comprising at least one inlaid elastic yarn (30),
b. at least two rows of stitches M1 and M2 (60,70,80,86), each comprising at least one pile yarn (40) forming knitting stitches, said at least one row of stitches T being disposed between adjacent rows M1 and M2, and **in that** the length of at least 80% by number of the pile strands (50) of said at least one pile yarn (40) in one of the rows of stitches M1 and M2 (60,70,80,86) is greater than or equal to the distance (d) separating the rows of stitches M1 and M2.

8. The compression garment (1) as claimed in any one of claims 1 to 7, **characterized in that** the length of at least 80% by number of the pile strands (50) per centimeter, measured over the length of the pile yarn (40), is greater than or equal to 1 mm, preferably greater than or equal to 5 mm, more preferably greater than or equal to 10 mm.

9. The compression garment (1) as claimed in any one of claims 1 to 8, **characterized in that** the pile yarn (40) is manufactured by knitting on a crochet knitting machine.

10. The compression garment (1) as claimed in any one of claims 1 to 9, **characterized in that** the pile yarn (40) forms knitted stitches, in particular tucked stitches and/or looped stitches.

11. The compression garment as claimed in any one of claims 1 to 10, **characterized in that** the knitted piece comprises an inlaid pile yarn.

12. The compression garment as claimed in any one of claims 1 to 11, **characterized in that** the elastic yarn comprises an elastic core and one or more cover yarn(s), and **in that** the pile yarn is a yarn selected from said cover yarn(s).

13. The compression garment as claimed in any one of claims 1 to 12, **characterized in that** the elastic yarn comprises an elastic core and one or more cover yarn(s), and **in that** the core has a titer less than or equal to 740 dtex.

14. The compression garment (1) as claimed in any one of claims 1 to 13, **characterized in that** the pile yarn (40) has a titer greater than or equal to 15 000 Nm, in particular greater than or equal to 20 000 Nm.

15. Use of a pile yarn (40), to facilitate slipping on and/or slipping off, in the knitting of a compression knitted piece (10) for the manufacture of a compression garment (1), said knitted piece (10) comprising at least one knitting ground yarn (20) and at least one elastic yarn, in particular inlaid yarn (30), said at least one pile yarn (40) is knitted in the knitted piece (10) so that the knitted piece (10) has, according to all or part of its inner face (12) and its outer face (14), pile strands (50) projecting from the latter, said pile strands (50) being arranged so as to be oriented in the direction in which the knitted piece (10) is slipped on (F1) and/or slipped off (F2).

16. Use as claimed in claim 15, **characterized in that** the knitted piece (10) is knitted on a circular knitting machine or on a flat knitting machine, and **in that** the pile yarn (40) is produced in a knitting step, in particular on a crochet knitting machine, prior to the step of knitting the knitted piece (10).
